Europäisches Patentamt

European Patent Office

Office européen des brevets

(19)

(11) Numéro de publication: **0 286 458**
A1

(12)

# DEMANDE DE BREVET EUROPEEN

(21) Numéro de dépôt: 88400436.7

(22) Date de dépôt: 25.02.88

(51) Int. Cl.⁴: **A 61 L 9/01**
A 61 L 9/12

(30) Priorité: 26.02.87 FR 8702555

(43) Date de publication de la demande:
12.10.88 Bulletin 88/41

(84) Etats contractants désignés:
AT BE CH DE ES FR GB GR IT LI LU NL SE

(71) Demandeur: Reckitt & Colman S.A.
15, rue Ampère
F-91301 Massy Cédex (FR)

(72) Inventeur: Sarsi, Patrick
27 avenue Ambroise Paré
F-28000 Chartres (FR)

Navier, Francis
53 rue des Comtesses
F-28000 Chartres (FR)

Chauffour, Dominique
12 Impasse du Clos Thiron
F-28630 Morancez (FR)

(74) Mandataire: Rinuy, Santarelli
14, avenue de la Grande Armée
F-75017 Paris (FR)

(54) Matériau-support perfectionné pour substance volatile applicable aux blocs désodorisants ou parfumants.

(57) Ce matériau-support est caractérisé en ce qu'il se présente sous la forme d'un solide dont la totalité ou une partie non négligeable de la surface extérieure diffusant ladite substance volatile présente des aspérités ou des rugosités apparentes augmentant notablement la surface développée de ce solide par rapport à la même surface lisse.

**Description**

## MATERIAU-SUPPORT PERFECTIONNE POUR SUBSTANCE VOLATILE APPLICABLE AUX BLOCS DESODORISANTS OU PARFUMANTS

La présente invention concerne un matériau-support perfectionné pour substances volatiles applicable aux blocs désodorisants ou parfumants exposés à des atmosphères humides ou à l'action de l'eau.

De façon plus particulière, l'invention concerne de tels matériaux-supports pour diffuser la substance volatile, du type parfum, applicables notamment aux blocs désodorisants ou parfumants suspendus en atmosphère humide, tels que les blocs pour salles de bains et pour l'assainissement des cuvettes de W.C.

De tels blocs et les dispositifs de suspension les contenant sont bien connus pour l'assainissement d'atmosphères exposées à l'humidité et à l'action de l'eau, comme c'est le cas de blocs suspendus au bord des cuvettes de W.C. De tels dispositifs pour W.C. affectant généralement la forme de paniers parallélépipédiques ou cylindriques à parois grillagées ou munies de fentes ou de perforations sont soumis à l'action de l'humidité ou de l'eau qui dissolvent et/ou entraînent avec elles les constituants nécessaires soit à une opération de nettoyage, soit à une action désinfectante, parfumante ou désodorisante, suivant la nature de ces constituants.

Généralement, et dans le cas particulier des compositions de traitement des cuvettes de W.C., les constituants des blocs en question comprennent (a) un agent de détartrage qui peut être un composé ou un mélange de composés évitant l'accumulation du tartre ou facilitant l'élimination de ce dernier ; (b) éventuellement un ou plusieurs surfactifs qui agissent en tant qu'agents détergents et/ou moussants ; (c) un ou plusieurs sels favorisant l'action du ou des agents surfactifs -dits "builders"- et, (d) une substance volatile du type désodorisant ou parfum se libérant dans la plupart des cas du sein du bloc par évaporation superficielle et par dissolutions successives de chacun des constituants de la composition de ce bloc.

L'espace compris entre le rebord d'une cuvette de W.C. et cette cuvette elle-même est relativement restreint. De plus, certains utilisateurs considèrent la vue du dispositif contenant ce bloc comme inesthétique.

Pour éviter ces inconvénients, la réalisation de ces dispositifs doit être telle que leurs formes et leurs dimensions conduisent à un volume le plus réduit possible, compatible avec les buts visés. La place disponible pour loger la composition de traitement est donc limitée.

En outre, pour que le bloc puisse satisfaire pleinement les besoins et les désirs du consommateur, il faut qu'il ait une durée d'action convenable. Ainsi, des modifications ont été apportées aux compositions de traitement des cuvettes de W.C. de manière à prolonger cette durée d'action. C'est ainsi, par exemple, que l'on a déjà prévu des enrobages ayant pour but de retarder la libération des constituants de ces compositions. De même, ces dernières peuvent renfermer des constituants présentant de faibles taux de solubilité ou vitesse de dissolution.

De telles modifications ne sont applicables que dans la limite où elles ne rendent pas la composition inefficace par une trop forte insolubilisation. Par voie de conséquence, l'émission du parfum peut être contrariée.

Or, on sait imprégner des matériaux absorbants au moyen de compositions parfumantes ou désodorisantes. Ces matériaux se présentent par exemple sous forme de plaques que l'on suspend dans des locaux ou autres enceintes pour en assainir ou en parfumer l'atmosphère.

L'efficacité de tels systèmes est fonction de la quantité de la substance volatile qui a été absorbée par le matériau absorbant et/ou du taux de diffusion de cette substance volatile.

La présente invention vise à améliorer ce taux de diffusion tout en faisant appel à un matériau absorbant se présentant sous une forme et un encombrement le plus faible possible. De plus, l'invention vise un tel matériau applicable au traitement des cuvettes de W.C., de manière que la durée de ce traitement soit prolongée.

Ainsi, conformément à l'invention, celle-ci fournit un matériau absorbant se présentant sous une forme solide et dont la totalité ou une partie non négligeable de la surface extérieure présente des aspérités et/ou des rugosités, ce matériau absorbant étant imprégné d'une substance volatile. Un tel matériau peut être appliqué seul, en association avec, ou incorporé à un bloc d'assainissement des cuvettes de W.C.

Le matériau absorbant peut être tout matériau poreux sous forme d'un mat, d'une plaque ou d'une feuille plane ou courbe ou d'un bloc avantageusement parallélépipédique ou cylindrique. Ces formes peuvent également être obtenues à partir de matériaux tissés ou non-tissés. En effet, elles peuvent être réalisées à partir de fibres ou de poudres incorporées dans une matrice appropriée par frittage, collage, par fusion superficielle ou enrobage formant ainsi des éléments de grandes dimensions que l'on peut, si nécessaire, découper ensuite à la dimension voulue.

Suivant une variante, le matériau selon l'invention peut se présenter sous la forme d'un matériau absorbant particulaire, par exemple un matériau granulaire à granulométrie relativement élevée et à structure alvéolaire.

La Demanderesse a constaté qu'une quantité minime (par unité de temps) de substance volatile telle qu'un parfum, diffuse dans l'atmosphère dans le cas d'un support de surface totale relativement faible, conduisant ainsi à une odorisation insuffisante de l'atmosphère d'un cabinet de W.C. ou d'une salle de bains.

Or, un matériau conforme à l'invention et se présentant sous les formes ci-dessus comportant superficiellement des zones rugueuses ou à aspérités, constitue un élément diffusant une quantité plus importante de parfum en raison même de la présence de ces aspérités et/ou rugosités superficielles.

Suivant une forme de réalisation avantageuse, le matériau peut comporter sur la totalité ou une partie de sa surface apparente de nombreuses rayures ou cannelures. Cette surface peut également être gaufrée.

Suivant un autre mode de réalisation, les aspérités et/ou rugosités sur la totalité ou une partie de la surface apparente peuvent être réalisées par des éléments particuliers rapportés sur la totalité ou une partie de la surface lisse initiale du matériau, cet apport de ces éléments se faisant par une opération visant à faire adhérer ces éléments sur ladite ou lesdites surface(s) initialement lisse(s).

Bien que la nature de la matière constitutive du matériau conforme à l'invention puisse être quelconque, à la seule condition qu'elle soit poreuse et susceptible d'absorber une substance volatile telle qu'un parfum, certaines matières ainsi imprégnées se sont avérées capables de retenir une certaine quantité de substance volatile pendant une durée relativement plus longue que d'autres matières imprégnées et ce, en présence d'eau.

De façon avantageuse, la Demanderesse a trouvé que les matières naturellement hydrophobes sont les matières donnant le meilleur résultat.

On pense que lorsqu'un matériau imprégné a rapidement perdu la quantité de substance volatile dont il a été imprégné, cette perte est d'abord due à l'évaporation ou à la diffusion de cette substance volatile mais que, par contre, cette perte de substance volatile peut être également due à l'entraînement par l'eau, au contact de laquelle se trouve le matériau, lorsqu'il est utilisé sous les rebords des cuvettes de W.C. au même titre que les autres constituants d'une composition généralement utilisée pour l'assainissement de ces cuvettes.

En choisissant alors, et ceci suivant un mode de réalisation encore plus avantageux, un matériau-support tel que la tension interfaciale du couple substance volatile-support est inférieure à la tension interfaciale du couple eau-support, la perte de substance volatile au contact de l'eau est réduite au minimum.

Par conséquent, la présente invention fournit aussi un matériau sous forme de mats, feuilles, plaques ou masse de particules agglomérées d'origine naturelle ou synthétique de nature organique ou minérale présentant les caractéristiques de tension de surface ci-dessus ou même ne les présentant pas naturellement mais dont au moins une partie de la surface apparente a été avantageusement modifiée par voie physico-chimique pour lui conférer ces caractéristiques par un traitement analogue à celui décrit dans la demande de brevet français N° 82.03599.

Parmi ces matières, on citera entre autres les polyoléfines comme le polyéthylène et le polypropylène, les polyesters, les polyamides, les esters de cellulose comme les acétate, propionate et butyrates de cellulose ou les mélanges et combinaisons de ceux-ci, les dérivés polyvinyliques comme le chlorure, l'acétate ou le phtalate de polyvinyle, les polyuréthannes sous forme expansée rigides et souples, leurs copolymères et mélanges, les fibres de verre éventuellement revêtues d'une pellicule de polyesters et analogues.

Parmi les matières d'origine naturelle, on citera la laine, en particulier. sous forme de feutres. Ces matières peuvent se présenter sous forme de particules ou de fibres. Ces fibres sont éventuellement transformées en feuilles ou en faisceaux par des méthodes connues telles que tissage, tricotage, superposition de couches, encollage et autres procédés de fabrication d'agglomérés ou de stratifiés, etc.

Bien entendu, le matériau selon l'invention peut être réalisé en grande surface, plaques, panneaux, etc. susceptibles d'être ultérieurement découpés en bandes, feuilles, plaques de petites dimensions pour être utilisés selon l'invention.

Les agglomérés obtenus à partir d'éléments particulaires peuvent l'être par exemple par frittage, éventuellement en présence de solvants, de manière à réaliser des produits absorbants. Là encore de telles structures sont susceptibles d'être découpées en bandes, feuilles ou blocs ou éléments particulaires et façonnés de manière à augmenter l'aire de leur diffusion pour être utilisés selon l'invention.

Les aspérités sur la totalité ou une partie de la surface apparente des matériaux selon l'invention peuvent être obtenues par la formation de cannelures, de rainures, d'arêtes, de creux, d'ondulations, etc.

Ces modifications de surface peuvent être réalisées sur un matériau existant déjà. Etant donné que le taux d'absorption dépend de la surface réelle, lorsque l'imprégnation est effectuée par immersion dans le fluide considéré, il peut être avantageux dans ce cas que le matériau à imprégner ait déjà sa ou ses surface(s) modifiée(s) avant de commencer cette imprégnation. Comme le but poursuivi est d'augmenter le taux de libération ou la diffusion de la composition volatile en cours d'utilisation pour désodoriser ou parfumer une atmosphère, la modification de surface peut aussi être effectuée, si on le désire, après l'opération d'imprégnation.

Un tel procédé peut toutefois comporter le risque d'une perte de substance volatile au cours des opérations de découpe ou de façonnage.

L'imprégnation du substrat constitué du matériau sous forme de particules, de nappes, de feuilles ou de plaques par une composition de parfum ou de désodorisant appropriée contenant éventuellement un solvant peut être effectuée par des techniques classiques, par exemple par immersion, par pulvérisation ou par injection au sein de la masse à imprégner ou par tous autres moyens appropriés. De telles opérations visent à assurer que la quantité voulue de la composition volatile par unité de surface ou par unité de poids du matériau à imprégner soit bien absorbée. Lorsqu'on utilise un solvant, ce dernier peut rester lui-même absorbé ou bien il peut être ultérieurement éliminé, si on le désire, par une opération d'évaporation. On prendra soin toutefois de choisir le solvant approprié et les composants de la substance parfumante ou désodorisante de manière à éviter des phénomènes d'incompatibilité avec le matériau absorbant.

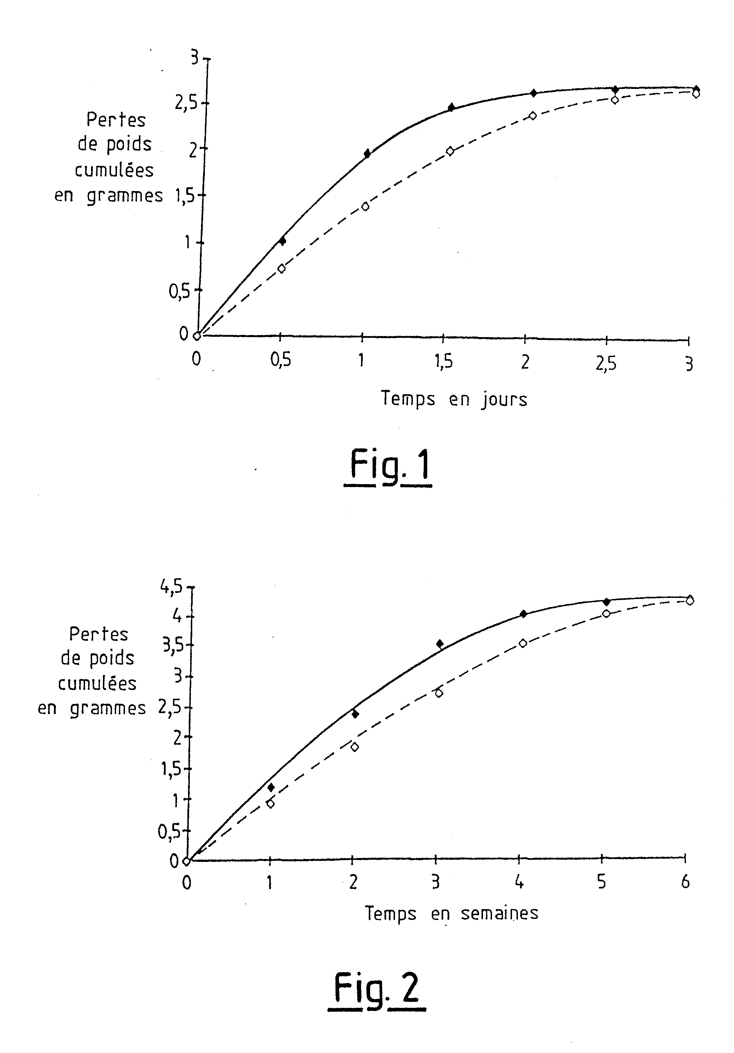

La portée et l'intérêt de l'invention ressortiront plus clairement des exemples donnés ci-après, avec référence aux figures 1 à 7 annexées qui sont des courbes d'évaporation suivant les cas envisagés ci-après.

Exemple 1

Une plaquette absorbante rigide constituée de fibres de cellulose naturelle de bois, de forme parallélépipédique et de dimensions 70 X 32 X 4 mm, a été fraisée sur ses deux faces principales dans le sens de la plus grande dimension, de façon à obtenir 12 cannelures parallèles, de section carrée, de 1 mm de largeur et de profondeur, à un intervalle de 2.5 mm.

La surface développée de chaque face principale a donc été augmentée de 75 % par rapport à la même face lisse, ce qui se traduit par une augmentation de la surface totale de la plaquette de 60 %.

La plaquette à faces cannelées ainsi qu'une plaquette lisse de mêmes dimensions ont été imprégnées avec 3 grammes d'acétate d'isobornyle puis placées dans un local à température et humidité contrôlées. L'évaporation de l'acétate d'isobornyle qui se traduit par une diminution du poids des plaquettes a été suivie par des pesées à intervalles réguliers.

Les pertes de poids cumulées de chaque plaquette ont été reportées sous forme de courbes $\Delta P = f(t)$ dans la figure 1.

La pente de ces courbes en un point donné représente la vitesse d'évaporation de l'acétate d'isobornyle en grammes par jour, vitesse à laquelle est proportionnelle l'intensité d'odorisation de la plaquette considérée.

La perte de poids de la plaquette à faces cannelées (courbe continue) est plus rapide que celle de la plaquette à faces lisses (courbe discontinue).

Il apparait donc que l'évaporation de l'acétate d'isobornyle est plus rapide d'environ 50 % sur la plaquette à faces cannelées, ce qui prouve l'efficacité de l'augmentation de la surface de diffusion pour évaporer une même composition parfumante à partir de substrats de dimensions identiques, et donc pour augmenter l'intensité d'odorisation.

Exemple 2

La nature du substrat de cet exemple est principalement de l'acétate de cellulose possédant un degré d'estérification d'environ 2,5. Des plaquettes parallélépipédiques rigides de dimensions 70 X 32 X 5 mm ont été préparées à partir d'un faisceau de fibres d'acétate de cellulose liées entre elles grâce à l'utilisation d'une faible quantité d'un solvant approprié, puis pressées de façon à former une nappe compacte qui est ensuite coupée à la longueur désirée.

Le procédé de fabrication permet d'obtenir des plaquettes à faces principales lisses ou possédant divers reliefs, donc une surface développée supérieure à la surface apparente.

La plaquette à surface de diffusion augmentée de cet exemple a été obtenue par pressage du faisceau de fibres entre deux rouleaux pourvus de stries en relief de section triangulaire.

Après relaxation du matériau et coupe de la nappe à la longueur désirée de 70 mm, la plaquette possède sur ses deux faces principales des "joncs" équidistants parallèles à la direction du faisceau de fibres et de section approximativement semi-circulaire.

Leur hauteur est d'environ 0,6 mm et leur répartition sur la largeur de la plaquette est uniforme, de l'ordre de 6,7 joncs par centimètre (1,5 mm d'entraxe).

Dans cet exemple, les faces principales de la plaquette à surface modifiée ne sont striées que jusqu'à environ 3 mm de chacun des deux bords de 70 mm, laissant donc de part et d'autre de la zone striée deux bandes lisses de 3 mm de largeur, nécessaires pour des raisons techniques de manipulation des plaquettes.

Une plaquette striée conforme à la description ci-dessus possède une surface de diffusion supérieure d'environ 40 % à celle d'une plaquette à faces lisses.

Une telle plaquette striée et une plaquette lisse sont imprégnées avec 5 g d'une composition parfumée florale.

L'évaporation de la composition parfumée est suivie conformément à la méthode de l'exemple 1.

Les courbes de pertes de poids cumulées en fonction du temps reportées dans la figure 2 montrent que l'évaporation du parfum à partir de la plaquette à faces striées (courbe continue) est d'environ 30 % plus rapide que l'évaporation du même parfum à partir de la plaquette à faces lisses (courbe discontinue).

Exemple 3

Dans cet exemple sont comparés les comportements des deux substrats suivants, soumis et non soumis à l'action de l'eau :
- une plaquette à base d'acétate de cellulose de degré d'estérification voisin de 2,5, de dimensions 70 X 32 X 5 mm, à faces lisses, obtenue suivant la méthode décrite dans l'exemple 2 ;
- une plaquette à base de fibres de cellulose naturelle de bois, de dimensions 70 X 32 X 4 mm, à faces lisses, identique à celle de l'exemple 1.

Les plaquettes sur lesquelles ont été déposées 5 grammes de compositions parfumante florale sont à présent placées dans des dispositifs désodorisants d'ambiance destinés à être fixés sous le rebord des cuvettes de W.C.

Chaque dispositif est testé individuellement dans la cuvette de W.C. équipant chacune des cabines identiques de l'installation d'évaluation.

Les cabines d'un volume de 2,4 m³ -dimensions 0,8 X 1,2 X 2,5 m- sont toutes soumises aux mêmes conditions de température, d'humidité et de ventilation.

L'intensité de l'odorisation de ces cabines par les dispositifs désodorisants d'ambiance est évaluée au travers d'une fenêtre pratiquée dans leur porte, par les membres entraînés d'un panel d'olfaction, suivant le barème ci-après :

5 : intensité très forte,

4 : intensité forte,

3 : intensité moyenne/odeur facilement perceptible,

2 : intensité faible/odeur difficilement perceptible,

1 : intensité nulle/odeur non perceptible.

Par ailleurs, les chasses d'eau des cuvettes de W.C. peuvent être actionnées indépendamment par un système de programmation automatique simulant des conditions d'utilisation réelle des produits (par exemple 30 tirs de chasse d'eau par jour).

Quatre dispositifs désodorisants d'ambiance, deux par nature de substrat, sont placés dans les cuvettes de quatre cabines de test pendant six semaines. Pour chaque nature de substrate, un exemplaire est soumis à l'action de l'eau par le système de programmation des tirs de chasse d'eau, l'autre étant maintenu au sec en coupant le système de programmation de la cuvette.

Les quatre dispositifs sont désignés comme suit :

A : dispositif avec plaquette d'acétate de cellulose, soumis aux tirs de chasse d'eau,

B : dispositif identique à A, non soumis aux tirs de chasse d'eau,

C : dispositif avec plaquette de cellulose naturelle de bois, soumis aux tirs de chasse d'eau,

D : dispositif identique à C, non soumis aux tirs de chasse d'eau.

La figure 3 montre l'évolution de l'intensité de l'odorisation des cabines contenant les dispositifs A et B pendant six semaines, à raison d'une évaluation par semaine.

La figure 4 montre l'évolution obtenue dans les mêmes conditions avec les dispositifs C et D.

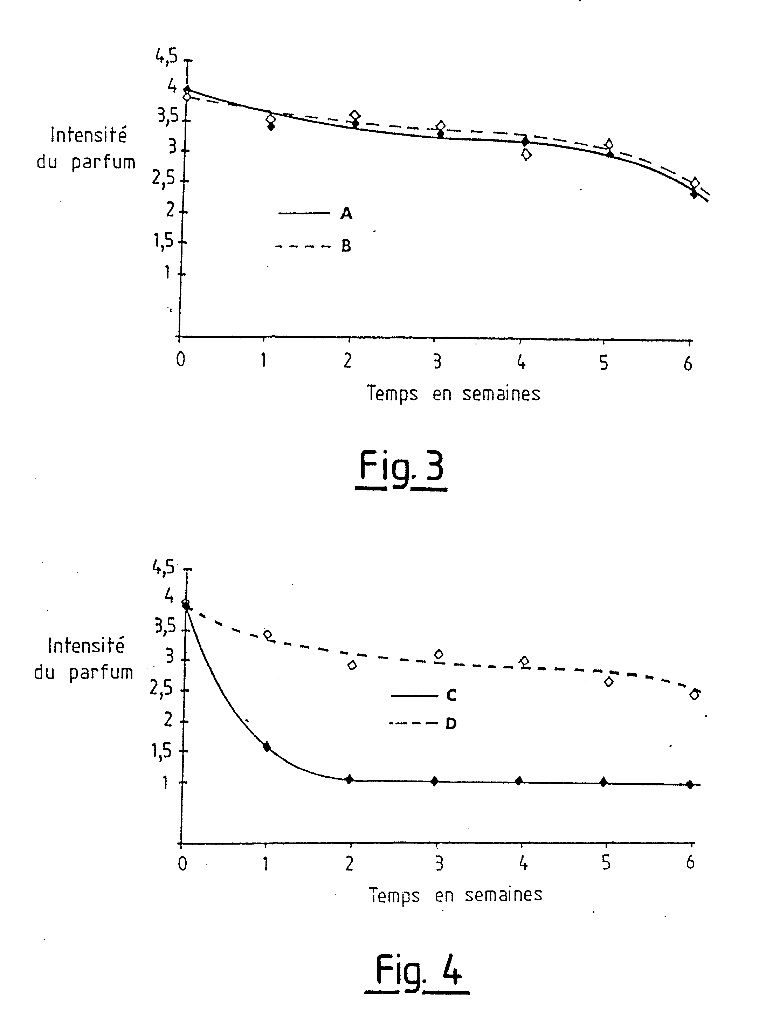

Sur la figure 3, les courbes d'intensité obtenues avec les dispositifs désodorisants A et B sont comparables pendant toute la durée du test, comprises entre environ 3,3 et 3,5, ce qui montre qu'il n'y a pas perte d'efficacité du dispositif désodorisant A soumis à l'action de l'eau par rapport à son homologue B maintenu au sec.

La composition parfumée n'est donc pas désorbée par l'eau lorsqu'elle est imprégnée sur de l'acétate de cellulose de degré d'estérification voisin de 2,5.

La figure 4 montre qu'en une semaine, l'intensité de l'odorisation de la cabine par le dispositif C soumis à l'action de l'eau chute au voisinage de la valeur de 1 alors que l'intensité du dispositif D maintenu au sec reste voisine de la valeur de 3 au-delà de cinq semaines.

La perte de rendement du dispositif C est de tout évidence due à la désorption rapide de la composition parfumée sous l'action de l'eau.

Un substrat de type cellulose de bois, sans aucun type de traitement spécifique, ne peut donc pas convenir pour les dispositifs désodorisants d'ambiance faisant l'objet de la présente invention.

## Exemple 4

Les tests de l'exemple 3 sont à présent effectués avec un substrat absorbant constitué de polyéthylène fritté. Deux plaquettes parallélépipédiques de dimensions 70 X 32 X 4 mm sont découpées dans une feuille de polyéthylène fritté de 4 mm d'épaisseur.

Ces plaquettes sur lesquelles ont été déposés 5 grammes de la même composition parfumée de type floral sont placées dans des dispositifs désodorisants d'ambiance E et F, identiques à ceux de l'exemple 3, chacun étant ensuite suspendu dans la cuvette de W.C. d'une cabine de test.

Pendant les six semaines du test, E est soumis aux tirs de chasse d'eau et F est maintenu au sec.

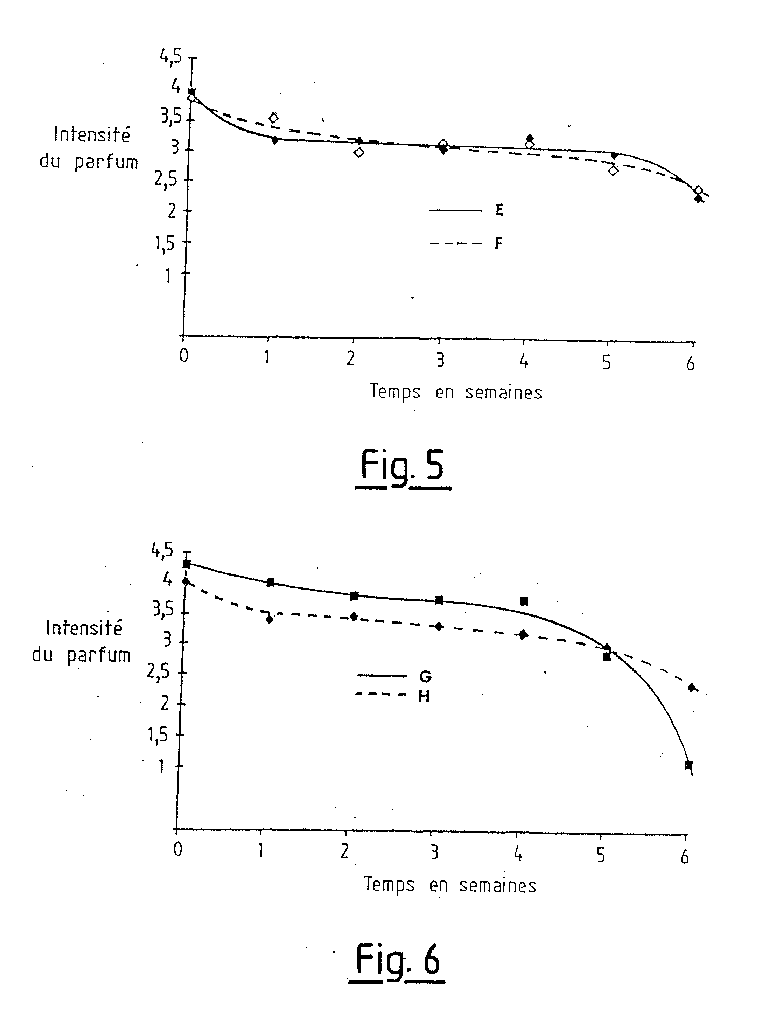

La figure 5 montre que les courbes d'intensité d'odorisation des deux dispositifs sont voisines et comprises entre 3,0 et 3,5 pendant toute la durée du test.

Ceci met donc en évidence l'aptitude du substrat à base de polyéthylène fritté à empêcher la désorption de la composition parfumée par l'eau.

## Exemple 5

Deux plaquettes d'acétate de cellulose, l'une striée, l'autre lisse, identiques à celles de l'exemple 2, imprégnées avec 5 grammes de la même composition parfumante florale, sont placées dans deux dispositifs désodorisants d'ambiance respectivement désignés G et H.

Ces dispositifs sont suspendus dans les cuvettes de W.C. de deux cabines d'olfaction et soumis à des aspersions répétées d'eau par le système de programmation automatique des tirs de chasse d'eau (30 tirs par jour).

La figure 6 montre l'évolution des intensités d'odorisation des cabines évaluées pendant six semaines suivant les critères de l'exemple 3.

Il apparait que l'intensité du dispositif désodorisant G équipé d'une plaquette striée, donc à plus grande surface de diffusion, est supérieure à l'intensité du dispositif désodorisant H équipé d'une plaquette lisse pendant environ cinq semaines.

Après cinq semaines, l'intensité du dispositif G tombe en-dessous de celle du dispositif H en raison de son épuisement plus rapide en composition parfumée (voir courbes de pertes de poids, exemple 2, figure 2).

En effet, il reste à ce moment davantage de composition parfumée dans le substrat du dispositif H du fait de l'évaporation plus lente au niveau de la plaquette lisse.

Cet exemple illustre donc bien l'accroissement de l'intensité d'odorisation obtenue dans des conditions d'utilisation réelle avec un substrat absorbant à surface de diffusion augmentée.

Corrélativement, à poids de composition parfumée égaux, la durée de vie utile d'un tel dispositif est plus courte que celle d'un dispositif comprenant un substrat à faces lisses.

## Exemple 6

Le test de l'exemple 5 est renouvelé avec le substrat absorbant de l'exemple 4 du type polyéthylène fritté.

Une plaquette parallélépipédique de ce matériau de dimensions 70 X 32 X 4 mm est rainurée dans le sens de la longueur sur ses deux faces principales avec un outil approprié.

Trente rainures de coupe en forme de triangle équilatéral de 1 mm de côté sont ainsi creusées côte à côte dans la plaquette.

L'aire de diffusion des faces principales de la plaquette est ainsi augmentée de près de 93 %.

Cette augmentation est d'environ 78 % pour la surface totale de la plaquette.

La plaquette ainsi rainurée et une plaquette à faces lisses, imprégnées avec 5 grammes de la même composition parfumante florale sont placées dans des dispositifs désodorisants d'ambiance respectivement J et K.

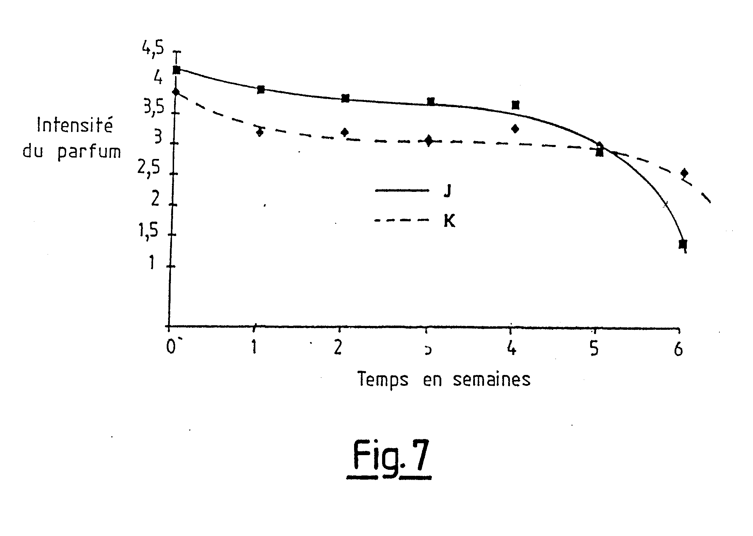

Après six semaines de test en cabines d'olfaction conformément à la procédure de l'exemple 5, les courbes d'intensité d'odorisation reproduites figure 7 montrent une nette supériorité du parfumage du dispositif J équipé d'une plaquette à surface de diffusion augmentée par rapport au dispositif K equipé d'une plaquette à faces lisses.

Dans une exécution préférée, un dispositif destiné à être accroché (suspendu) dans les cuvettes de W.C., contenant une composition active non parfumée de préférence sous forme d'un bloc et comprenant des agents détartrants et/ou des surfactifs et/ou un ou des "builder(s)", possède un logement (compartiment) pour un matériau absorbant qui se présente sous la forme d'un bloc mince imprégné de parfum du type de ceux utilisés dans les exemples ci-dessus. De tels supports absorbants possèdent une surface extérieure relativement importante en regard de leur épaisseur.

De plus, dans le cas d'un bloc mince, l'une au moins des plus grandes faces de ce bloc est façonnée de manière à présenter des crêtes et des sillons destinés à augmenter l'aire d'évaporation (de diffusion).

La hauteur (ou profondeur) relative des crêtes et des sillons représente environ 5 à 25 % de l'épaisseur du bloc et leur largeur varie en proportion.

De préférence, la dénivellation entre sommet de crête et fond de sillon représente de 8 à 20 % de l'épaisseur du bloc.

Pratiquement, le bloc possède des crêtes et sillons d'une hauteur (profondeur) allant de 10 à 15 % de l'épaisseur de la plaquette.

Une surface ainsi modifiée peut avoir entre 3 et 15 crêtes et 3 et 15 sillons par centimètre.

De préférence, la surface comporte de 4 à 12 crêtes et sillons par centimètre. Plus particulièrement, cette densité est comprise entre 5 et 10.

Ainsi, sous un relativement faible volume, mais grâce à son importante surface réelle, on obtiendra le maximum d'efficacité de diffusion de toute substance active en association à une composition d'assainissement.

Il va de soi que la présente invention n'a été décrite qu'à titre purement explicatif et nullement limitatif et que toute modification, notamment au niveau des équivalences techniques, pourra y être apportée sans sortir de son cadre.

**Revendications**

1. Matériau-support perfectionné pour substance volatile applicable aux blocs désodorisants ou parfumants exposés à des atmosphères humides ou à l'action de l'eau, caractérisé en ce qu'il se présente sous la forme d'un solide dont la totalité ou une partie non négligeable de la surface extérieure diffusant ladite substance volatile présente des aspérités et/ou des rugosités apparentes augmentant notablement la surface développée de ce solide par rapport à la même surface lisse.

2. Matériau-support selon la revendication 1, caractérisé en ce qu'il est choisi parmi les matériaux poreux et qu'il se présente sous la forme de mats, de plaques, de feuilles planes ou courbes, ou de blocs.

3. Matériau-support selon la revendication 1 ou 2, caractérisé en ce que la surface diffusante est discontinue.

4. Matériau-support selon l'une quelconque des revendications 1 à 3, caractérisé en ce que lesdites aspérités et/ou rugosités sont constituées par des rainures ou des cannelures.

5. Matériau-support selon l'une quelconque des revendications 1 à 3, caractérisé en ce que lesdites aspérités et/ou rugosités résultent de la saillie en surface d'une matrice de base, de fibres, granules ou particules.

6. Matériau-support selon la revendication 5, caractérisé en ce que lesdites fibres ou lesdits granules ou particules sont noyés dans la masse de ladite matrice ou sont portés à la superficie de cette dernière par collage, fusion ou frittage.

7. Matériau-support selon l'une quelconque des revendications 1 à 3, caractérisé en ce que ledit matériau consiste en un aggloméré de granules ou particules alvéolaires ou non dont tout ou partie de la surface a été rendue rugueuse.

8. Matériau-support selon l'une quelconque des revendications 1 à 7, caractérisé en ce que la matière constitutive dudit matériau présente une tension interfaciale du couple substance volatile- support inférieure à la tension interfaciale du couple eau-support.

9. Matériau-support selon la revendication 8, caractérisé en ce que la matière absorbante constitutive de base a été traitée par voie physico-chimique de manière à présenter finalement une tension interfaciale du couple substance volatile-support inférieure à la tension interfaciale du couple eau-support.

10. Matériau-support selon l'une quelconque des revendications 1 à 8, caractérisé en ce que la matière constitutive de base est choisie parmi les polyoléfines comme le polyéthylène et le polypropylène, les polyesters, les polyamides, les esters de cellulose comme les acétate, propionate et butyrates de cellulose ou les mélanges et combinaisons de ceux-ci, les dérivés polyvinyliques comme le chlorure, l'acétate ou le phtalate de polyvinyle, les polyuréthannes sous forme expansée, rigides ou souples, leurs copolymères et mélanges, les fibres de verre éventuellement revêtues d'une pellicule de polyesters et analogues.

11. Matériau-support selon l'une quelconque des revendications 1 à 8, caractérisé en ce que ledit matériau est à base de laine.

12. Application du matériau-support selon l'une quelconque des revendications 1 à 11 à l'odorisation des atmosphères humides ou des cuvettes de W.C.

13. Application du matériau-support selon la revendication 12, caractérisé en ce qu'il est associé à ou fait partie intégrante d'une plaque ou d'un bloc de composition assainissante comprenant entre autres des agents détartrants et/ou anti-tartre, et/ou un ou plusieurs surfactifs et/ou une ou plusieurs charges actives ou non, contenu dans un dispositif de suspension ou de fixation au bord d'une cuvette de W.C.

14. Application du matériau-support selon la revendication 13, caractérisé en ce qu'il est inséré dans un compartiment séparé dudit dispositif d'assainissement.

0286458

Fig. 1

Fig. 2

0286458

Fig.3

Fig. 4

0286458

Fig. 5

Fig. 6

0286458

Fig.7

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | EP-A-0 152 722  (SIPURO) --- | | A 61 L   9/01 |
| A | EP-A-0 177 255  (RECKITT & COLMAN) --- | | A 61 L   9/12 |
| A | FR-A-1 598 644  (R. ARIES) --- | | |
| A | FR-A-1 590 898  (FINANCIERE D'INVESTISSEMENT DU CENTRE) --- | | |
| A | DE-A-1 492 452  (SECTO) ----- | | |

DOMAINES TECHNIQUES
RECHERCHES (Int. Cl.4)

A 61 L   9/01
A 61 L   9/12

Le présent rapport a été établi pour toutes les revendications

| Lieu de la recherche | Date d'achèvement de la recherche | Examinateur |
|---|---|---|
| LA HAYE | 22-06-1988 | PELTRE CHR. |